# EUROPÄISCHE PATENTANMELDUNG

(11) **EP 4 759 270 A1**
(43) Veröffentlichungstag der Anmeldung: **17.06.2026**
(21) Anmeldenummer: 25217463.6
(22) Anmeldetag: 20.11.2025
(51) Int. Cl.: A61F 2/28, A61F 2/30, A61B 17/80, B33Y 80/00

(54) **IMPLANTAT FÜR EINE FLÄCHIGE BEHANDLUNG EINES KNOCHENDEFEKTS**

(30) Priorität: 11.12.2024 DE 102024137112
(71) Anmelder: Karl Leibinger Asset Management GmbH & Co. KG, 78570 Mühlheim an der Donau (DE)
(72) Erfinder: Aksu, Adem, 78054 VS-Schwenningen (DE); Schaarschmidt, Lena, 78532 Tuttlingen (DE)
(74) Vertreter: Schwamberger, Martin

(57) **Zusammenfassung**

Die Erfindung betrifft ein Implantat (I) für eine flächige Behandlung eines Knochendefekts, insbesondere eines Knochendefekts im Bereich des Thorax oder des Craniums, umfassend eine Gitterstruktur (GS) mit einer Oberseite (OS) und einer der Oberseite (OS) abgewandt liegenden Unterseite (US), auf welcher eine knochenseitige Befestigung des Implantats (I) vorzunehmen ist. Die Gitterstruktur (GS) weist mindestens einen ersten Tragabschnitt (TA1) auf, welcher aus einem ersten biokompatiblen Werkstoff besteht. Des Weiteren weist die Gitterstruktur (GS) zusätzlich zu dem mindestens einen ersten Tragabschnitt (TA1) noch mindestens einen zweiten Tragabschnitt (TA2) auf, welcher jeweils über eine im Vergleich zu dem mindestens einen ersten Tragabschnitt (TA1) geringere Steifigkeit verfügt, indem der mindestens eine zweite Tragabschnitt (TA2) aus einem zweiten biokompatiblen Werkstoff hergestellt ist, welcher eine geringere Werkstoffsteifigkeit aufweist als der erste Werkstoff.

## Beschreibung

Die Erfindung betrifft ein Implantat für eine flächige Behandlung eines Knochendefekts, insbesondere eines Knochendefekts im Bereich des Thorax oder des Craniums, umfassend eine flexible Gitterstruktur mit einer Oberseite und einer der Oberseite abgewandt liegenden Unterseite, auf welcher eine knochenseitige Befestigung des Implantats vorzunehmen ist, wobei die Gitterstruktur mindestens einen ersten Tragabschnitt aufweist, welcher aus einem ersten biokompatiblen Werkstoff besteht.

Im Bereich der Implantate sind Ausführungen bekannt, welche zur flächigen Behandlung von Knochendefekten zur Anwendung kommen. Häufig verfügt ein derartiges Implantat über eine Gitterstruktur, welche zumindest teilweise flexibel gestaltet ist und hierdurch eine Anpassung an unterschiedliche Formen ermöglicht. Derartig gestaltete Implantate können im Bereich des Thorax und hierbei insbesondere bei Rippenfrakturen anstelle von Rippenplatten angewendet werden, um durch eine großflächige Anordnung am Thorax auch Bereiche zwischen verschiedenen Rippen abzudecken. Durch die Gitterstruktur wird dabei bei gleichzeitigem Schutz innenliegender Organe eine ausreichende Flexibilität verwirklicht, um eine ausreichende Beweglichkeit des Thorax zu ermöglichen.

Darüber hinaus werden Implantate mit Gitterstruktur im Bereich des Craniums für die Behandlung von Schädeldefekten vorgesehen, wobei die zumindest teilweise flexibel gestaltete Gitterstruktur hier eine Anpassung des Implantats an gekrümmte Teile des Schädels ermöglicht und zudem eine ausreichende Elastizität sicherstellt. Häufig verfügt ein Implantat zur flächigen Behandlung dabei über einen netzartigen, die Gitterstruktur bildenden Tragabschnitt, welcher am Knochen befestigt wird und der Stabilisierung dient.

Aus der DE 197 46 396 A1 geht ein Implantat hervor, welches für eine flächige Behandlung von Knochendefekten im Bereich des Craniums vorgesehen ist. Das Implantat weist eine Gitterstruktur auf, welche sich netzartig aus mehreren, in sich geschlossenen und untereinander verbundenen Segmenten zusammensetzt. Die Gitterstruktur kann an einer Unterseite zur Fixierung von Knochenteilen oder für eine Überbrückung von Knochenfehlstellen in dem entsprechenden Bereich des Craniums befestigt werden, wobei für die Befestigung dabei durch jedes einzelne Segment eine Knochenschraube hindurchgeführt werden kann. Die untereinander verbundenen Segmente bilden dabei bei der Gitterstruktur einen Tragabschnitt, über welchen seitens der Knochenteile bzw. der Knochenfehlstelle eine Stabilisierung realisierbar ist. Die Gitterstruktur ist hierbei mittels Ätzen aus Titan hergestellt.

Ausgehend vom vorstehend beschriebenen Stand der Technik ist es nun die Aufgabe der vorliegenden Erfindung ein Implantat zu schaffen, über welches eine flächige Behandlung eines Knochendefekts vorgenommen werden kann, wobei bei Verwendung dieses Implantats zudem die Gefahr von Gewebeirritationen vermindert sein soll.

Diese Aufgabe wird ausgehend vom Oberbegriff des Anspruchs 1 in Verbindung mit dessen kennzeichnenden Merkmalen gelöst. Die hierauf folgenden, abhängigen Ansprüche geben jeweils vorteilhafte Weiterbildungen der Erfindung wieder. Weitere vorteilhafte Ausgestaltungen ergeben sich aus der Beschreibung sowie aus den Figuren.

Gemäß der Erfindung umfasst ein Implantat eine Gitterstruktur mit einer Oberseite und einer der Oberseite abgewandt liegenden Unterseite, auf welcher eine knochenseitige Befestigung des Implantats vorzunehmen ist. Die Gitterstruktur weist mindestens einen ersten Tragabschnitt auf, welcher aus einem ersten biokompatiblen Werkstoff besteht.

Das erfindungsgemäße Implantat ist für eine flächige Behandlung eines Knochendefekts vorgesehen, wobei es sich dabei insbesondere um einen Knochendefekt im Bereich des Thorax oder des Craniums handelt. Für diese flächige Behandlung ist das Implantat mit einer Gitterstruktur ausgestattet, welche bevorzugt flächig gestaltet ist. Dabei verfügt die Gitterstruktur über eine Oberseite und eine Unterseite, welche einander abgewandt liegen und von denen es sich bei der Unterseite um die Seite handelt, mit welcher das Implantat auf den Knochendefekt zugewandt aufgelegt und befestigt wird.

Unter einer "flächigen" Behandlung ist im Sinne der Erfindung insbesondere zu verstehen, dass über das Implantat bei Anwendung an einem Knochendefekt eine Behandlungsfläche abgedeckt wird. Im Rahmen der Erfindung kann das Implantat konkret dafür vorgesehen sein, bei dieser flächigen Behandlung dabei Knochensegmente eines frakturierten Knochens zu verbinden und/oder Verbindungen zwischen unterschiedlichen Knochen herzustellen und/oder Knochenfehlstellen zu überdecken. Eine durch das erfindungsgemäße Implantat abzudeckende Behandlungsfläche kann dabei je nach konkretem Anwendungsfall, also insbesondere ob eine Anwendung im Bereich des Thorax oder des Craniums vorgesehen ist, im Bereich von wenigen Quadratmillimetern bis hin zu mehreren Quadratzentimetern liegen.

Die Erfindung umfasst nun die technische Lehre, dass die Gitterstruktur zusätzlich zu dem mindestens einen ersten Tragabschnitt noch mindestens einen zweiten Tragabschnitt aufweist, welcher jeweils über eine im Vergleich zu dem mindestens einen ersten Tragabschnitt geringere Steifigkeit verfügt, indem der mindestens eine zweite Tragabschnitt aus einem zweiten biokompatiblen Werkstoff hergestellt ist, welcher eine geringere Werkstoffsteifigkeit aufweist als der erste Werkstoff.

Mit anderen Worten setzt sich die Gitterstruktur des erfindungsgemäßen Implantats also aus Tragabschnitten mit voneinander abweichenden Steifigkeiten zusammen, indem in der Gitterstruktur neben dem mindestens einen ersten Tragabschnitt noch mindestens ein zweiter Tragabschnitt vorgesehen ist, welcher im Vergleich zu dem mindestens einen ersten Tragabschnitt mit einer geringeren Steifigkeit ausgeführt ist. Der mindestens eine erste Tragabschnitt besteht dabei aus einem ersten biokompatiblen Werkstoff und der mindestens eine zweite Tragabschnitt aus einem zweiten biokompatiblen Werkstoff, wobei die niedrigere Steifigkeit des mindestens einen zweiten Tragabschnitts im Vergleich zu dem mindestens einen ersten Tragabschnitt dadurch erreicht ist, dass der zweite Werkstoff eine niedrigere Werkstoffsteifigkeit aufweist als der erste Werkstoff.

Eine derartige Ausgestaltung eines Implantats hat dabei den Vorteil, dass durch den abschnittsweisen Aufbau der Gitterstruktur aus Tragabschnitten unterschiedlicher Steifigkeit eine optimale Anpassung des Implantats an die Erfordernisse der jeweiligen Behandlung möglich ist. So kann über den mindestens einen ersten, steiferen Tragabschnitt lokal gezielt eine hohe Stabilität verwirklicht werden, während über den mindestens einen zweiten Tragabschnitt aufgrund seiner im Vergleich zum ersten Tragabschnitt geringeren Steifigkeit lokal eine höhere Flexibilität der Gitterstruktur realisierbar ist, wodurch die Gitterstruktur von ihrer Form her einfacher an einen knochenseitig vorgegebenen Auflagebereich angepasst werden kann. Somit können bei dem erfindungsgemäßen Implantat durch Aufbau der Gitterstruktur aus den beiden Arten von Tragabschnitten gezielt unterschiedliche Bereiche geschaffen werden, nämlich mindestens ein Bereich hoher Stabilität aufgrund der höheren Steifigkeit des jeweiligen ersten Tragabschnitts und mindestens ein Bereich höherer Flexibilität und Nachgiebigkeit aufgrund der niedrigeren Steifigkeit des jeweiligen zweiten Tragabschnitts. Die voneinander abweichenden Steifigkeiten können dabei zuverlässig erreicht werden, indem die Tragabschnitte aus den, unterschiedliche Werkstoffsteifigkeiten aufweisenden Werkstoffen hergestellt sind. Darüber hinaus besteht die Möglichkeit, an den Tragabschnitten in Abhängigkeit des jeweils gewählten Werkstoffs weitere werkstoffabhängige Eigenschaften darzustellen.

Unter der "Steifigkeit" des jeweiligen Tragabschnitts ist im Sinne der Erfindung insbesondere der Widerstand des jeweiligen Tragabschnitts gegen eine durch äußere Belastung bewirkte Verformung zu verstehen. Mit "Werkstoffsteifigkeit" des jeweiligen Werkstoffs ist bevorzugt eine werkstoffmechanische Eigenschaft zu verstehen, welche durch das Verhältnis der wirkenden Spannung und der sich hierdurch einstellenden Dehnung definiert ist, wobei hierbei als Materialkennwerte insbesondere der Elastizitäts- und der Schubmodul des jeweiligen Werkostoffs relevant sind.

Aufgrund ihres abschnittsweisen Aufbaus ist die Gitterstruktur zumindest im Bereich des mindestens einen zweiten Tragabschnitts flexibel gestaltet, wodurch der Gitterstruktur insbesondere eine 3-dimensionale Formbarkeit verliehen wird, um problemlos eine Anpassung der Form des Implantats an einen knochenseitig vorgegebenen Auflagebereich vornehmen zu können. So ist die Gitterstruktur des erfindungsgemäßen Implantats durch ihre flexible Gestaltung insbesondere an einen gekrümmten Knochendefekt sowie einen Knochendefekt mit unregelmäßiger Form anpassbar. Bevorzugt ist die Gitterstruktur aber auch im Bereich des mindestens einen ersten Tragabschnitts formbar, wobei der mindestens eine erste Tragabschnitt Verformungen aufgrund seiner höheren Steifigkeit im Vergleich zum mindestens einen zweiten Tragabschnitt jeweils einen höheren Widerstand entgegensetzt. Allgemein kann die Gitterstruktur im Rahmen der Erfindung dazu ausgestaltet sein, durch den jeweils behandelnden Chirurgen von ihrer Größe und Form her durch Entfernen von Teilen der Gitterstruktur und damit von Teilen der Tragabschnitte angepasst zu werden, beispielsweise durch Zuschneiden.

Das erfindungsgemäße Implantat ist insbesondere dazu vorgesehen, bei der Behandlung des jeweiligen Knochendefekts knochenseitig eine Stabilisierung herbeizuführen. Dazu wird die Gitterstruktur bevorzugt im Bereich des zu behandelnden Knochendefekts knochenseitig befestigt, wobei diese knochenseitige Befestigung dabei in Form einer Befestigung an mehreren Knochensegmenten eines Knochens oder an mehreren Bereichen eines Knochens oder auch an unterschiedlichen Knochen vorliegen kann. Bevorzugt ist die Gitterstruktur dafür mit mehreren Befestigungspunkten ausgestattet, welche insbesondere in Form je eines Anschraubpunkts vorliegt, an dem die Befestigung des Implantats über jeweils eine Knochenschraube realisierbar ist. Insbesondere ist jeder der Tragabschnitte mit mehreren derartigen Befestigungspunkten versehen. Je nach konkretem Behandlungsfall und Größe der Behandlungsfläche kann sich die Gitterstruktur aus einem oder mehreren ersten Tragabschnitten sowie einem oder mehreren zweiten Tragabschnitten zusammensetzen.

Entsprechend einer Ausführungsform der Erfindung ist der mindestens eine zweite Tragabschnitt in der Gitterstruktur zwischen zumindest zwei ersten Tragabschnitten angeordnet. Der mindestens eine zweite Tragabschnitt koppelt die zumindest zwei ersten Tragabschnitte miteinander. In vorteilhafter Weise kann hierdurch über den mindestens einen zweiten Tragabschnitt zwischen mehreren steifen Tragabschnitten ein flexiblerer Zwischenbereich ausgestaltet werden, in welchem eine bessere Anpassbarkeit der Form des Implantats gegeben ist und in dem niedrigere Gewebeirritationen von angrenzendem Gewebe hervorgerufen werden. So können über die ersten Tragabschnitte gezielt Knochenbereiche stabilisiert und über den zwischenliegenden, zweiten Tragabschnitt zudem eine Koppelung dieser stabilisierten Knochenbereiche untereinander herbeigeführt werden. Dabei ist aufgrund der niedrigeren Steifigkeit des zweiten Tragabschnitts die Gefahr von Gewebeirritationen gezielt lokal abgesenkt und dennoch eine gewisse Beweglichkeit der stabilisierten Knochenbereiche zueinander geschaffen. Bei Anwendung des Implantats im Bereich des Thorax kann hierdurch beispielsweise eine gezielte lokale Erhöhung der Flexibilität des Implantats realisiert werden, um die ständige Bewegung des Thorax aufgrund der Atmung oder sonstiger Bewegungen des Patienten zu erleichtern.

Gemäß einer Ausgestaltungsmöglichkeit der Erfindung ist die Gitterstruktur modular aufgebaut, indem der mindestens eine erste Tragabschnitt und der mindestens eine zweite Tragabschnitt aneinander befestigt sind. Bevorzugt ist diese Befestigung dabei durch eine stoffschlüssige Verbindung zwischen den Tragabschnitten realisiert. In Weiterbildung dieser Ausgestaltungsmöglichkeit überdeckt der mindestens eine zweite Tragabschnitt im Bereich der jeweiligen Befestigung in einer quer zu der Oberseite und der Unterseite verlaufenden Richtung den mindestens einen ersten Tragabschnitt. **In** vorteilhafter Weise kann durch diese Überdeckung eine höhere Belastbarkeit der Verbindung zwischen den Tragabschnitten geschaffen werden. Ganz besonders bevorzugt umgreift der mindestens eine zweite Tragabschnitt den mindestens einen ersten Tragabschnitt im Bereich der jeweiligen Befestigung beidseitig mit vorstehenden Verbindungssegmenten, wodurch die Belastbarkeit der Verbindung weiter erhöht wird.

Es ist eine weitere Ausführungsform der Erfindung, dass zumindest einer der Tragabschnitte je eine Netzstruktur aufweist, welche durch miteinander über Zwischensegmente verbundene, in sich geschlossene Segmente gebildet ist. Hierdurch wird bei dem jeweiligen Tragabschnitt ein Aufbau verwirklicht, bei welchem eine Beweglichkeit und damit Flexibilität im Bereich des jeweiligen Tragabschnitts geschaffen ist. Bevorzugt sind die in sich geschlossenen Segmente dabei ringförmig gestaltet, wobei die Segmente aber im Rahmen der Erfindung auch hiervon abweichende Gestaltungen aufweisen können, beispielsweise jeweils in Form je eines Polygons. Die Segmente sind bei der Netzstruktur dabei über Zwischensegmente miteinander verbunden, die linear oder auch nichtlinear gestaltet sein können. Denkbar wäre zudem auch, dass die Zwischensegmente durch Befestigung aneinander je ein geschlossenes Segment definieren. Vorliegend können entweder der mindestens eine erste Tragabschnitt oder der mindestens eine zweite Tragabschnitt oder sowohl der mindestens eine erste Tragabschnitt als auch der mindestens eine zweite Tragabschnitt jeweils mit einer derartigen Netzstruktur versehen sein.

Alternativ oder ergänzend dazu ist zumindest einer der Tragabschnitte plattenförmig gestaltet. Ganz besonders bevorzugt ist eine plattenförmige Gestalt dabei bei dem mindestens einen ersten Tragabschnitt vorgenommen, um hier eine höhere Steifigkeit zu erreichen. Prinzipiell könnte aber auch der mindestens eine zweite Tragabschnitt plattenförmig ausgeführt sein.

In Weiterbildung der Erfindung ist zumindest einer der Tragabschnitte mittels eines additiven Fertigungsverfahrens hergestellt. Dabei kommt im Sinne der Erfindung insbesondere eine Fertigung im Rahmen eines 3D-Druckverfahrens infrage. Besonders bevorzugt ist der mindestens eine erste Tragabschnitt durch additive Fertigung, insbesondere durch 3D-Druck, ausgestaltet worden.

Weiter bevorzugt weist der zweite biokompatible Werkstoff eine geringere Härte auf als der erste Werkstoff. Dies hat den Vorteil, dass damit der mindestens eine zweite Tragabschnitt weicher gestaltet ist als der mindestens eine erste Tragabschnitt, wodurch die Gefahr von Gewebeirritationen im Bereich des jeweiligen zweiten Tragabschnitts reduziert wird.

Gemäß einer Ausgestaltungsmöglichkeit der Erfindung ist der erste Werkstoff ein Metall oder eine Metalllegierung, insbesondere Titan oder eine Titanlegierung. Ganz besonders bevorzugt handelt es sich bei dem ersten Werkstoff aber um einen Kunststoff, wobei dieser Kunststoff dabei insbesondere ein Polymer, bevorzugt ein Thermoplast und besonders bevorzugt Polyetheretherketon (PEEK) ist. Denn insbesondere Polyetheretherketon zeichnet sich durch eine sehr gute Biokompatibilität und eine hohe Werkstoffsteifigkeit aus, so dass über den jeweiligen ersten Tragabschnitt damit auch eine hohe Stabilität der Gitterstruktur dargestellt werden kann.

Der zweite Werkstoff ist vorzugsweise ein Kunststoff, insbesondere ein Polymer, bevorzugt ein Thermoplast und besonders bevorzugt Polyethylen (PE) , beispielsweise ultrahochmolekulargewichtiges Polyethylen (UHMWPE) oder Polyethylen hoher Dichte (HDPE). Hierdurch kann die im Vergleich zu dem jeweiligen ersten Tragabschnitt darzustellende, niedrigere Steifigkeit des jeweiligen zweiten Tragabschnitts auf zuverlässige Weise erreicht werden. Insbesondere bildet das Polyethylen den jeweiligen zweiten Tragabschnitt dabei als nicht poröse Schicht aus, wodurch trotz der niedrigeren Steifigkeit eine ausreichende Stabilität erreicht und zudem eine glatte Oberfläche des jeweiligen zweiten Tragabschnitts dargestellt werden kann.

Ganz besonders bevorzugt sind die beiden vorgenannten Varianten gemeinsam verwirklicht, wobei zur Herstellung des erfindungsgemäßen Implantats zunächst die Herstellung des mindestens einen ersten Tragabschnitts aus Polyetheretherketon (PEEK) vorgenommen wird. Dies findet dabei besonders bevorzugt im Rahmen einer additiven Fertigung statt. Anschließend wird zur Reinigung bevorzugt eine Plasmabehandlung/Plasmaaktivierung des mindestens einen ersten Tragabschnitts durchgeführt, bevor der mindestens eine erste Tragabschnitt gemeinsam mit Polyethylen-Pulver schichtweise in einer Negativform platziert wird. In einem darauffolgenden Pressvorgang wird das Polyethylen (PE) dann zusammen mit dem Polyetheretherketon (PEEK) erhitzt, wodurch das Polyethylen mit dem Polyetheretherketon verschmilzt und hierbei den mindestens einen zweiten Tragabschnitt bildet. Hierdurch lässt sich ein belastbarer Verbund zwischen Polyetheretherketon und Polyethylen erreichen.

Es ist eine weitere Ausgestaltungsmöglichkeit der Erfindung, dass auf zumindest einen der Tragabschnitte zumindest abschnittsweise eine Deckschicht aufgebracht ist, welche im Vergleich zu dem jeweiligen Tragabschnitt eine geringere Härte aufweist. Durch das zumindest abschnittsweise Aufbringen einer solchen Deckschicht auf den jeweiligen Tragabschnitt und die im Vergleich zu diesem Tragabschnitt geringere Härte dieser Deckschicht kann die Gefahr von Haut- oder Gewebeirritationen vermindert werden. Denn durch das zumindest abschnittsweise Abdecken des jeweiligen Tragabschnitts mit der jeweiligen Deckschicht wird in dem jeweiligen Bereich aufgrund der niedrigeren Härte der Deckschicht eine weichere Oberfläche des Implantats verwirklicht.

Insbesondere ist die Deckschicht dabei zumindest auf den mindestens einen ersten Tragabschnitt aufgebracht. Dies ist bevorzugt dann verwirklicht, wenn der mindestens eine erste Tragabschnitt im Rahmen eines additiven Fertigungsverfahrens hergestellt worden ist. Dadurch können über die Deckschicht Unebenheiten und Kanten dieses Tragabschnitts überdeckt und damit in diesem Bereich eine weichere Oberfläche des Implantats geschaffen werden.

Im Sinne der Erfindung ist unter "Härte" der mechanische Widerstand zu verstehen, der einem mechanischen Eindringen entgegengesetzt wird. Dementsprechend setzt also die Deckschicht einem mechanischen Eindringen einen geringeren mechanischen Widerstand entgegen als der jeweilige Tragabschnitt. Die Deckschicht kann also im Vergleich zu dem jeweiligen Tragabschnitt auch als weicher beschrieben werden.

Bei der vorgenannten Ausgestaltungsmöglichkeit ist der jeweilige Tragabschnitt zumindest abschnittsweise mit der Deckschicht beschichtet, d.h. der jeweilige Tragabschnitt kann also an einem oder mehreren Abschnitten oder auch vollumfänglich mit der jeweils einen Deckschicht versehen sein.

Entsprechend einer Ausführungsform der Erfindung ist der jeweilige Tragabschnitt auf der Oberseite der Gitterstruktur an mindestens einem Teilabschnitt mit der jeweils Deckschicht beschichtet. In diesem Fall kann die Deckschicht also auf den jeweiligen Tragabschnitt abschnittsweise auf der Oberseite aufgebracht sein, wodurch der jeweilige Tragabschnitt dann an der Oberseite abschnittsweise unbeschichtet ist. Alternativ dazu ist die Beschichtung des jeweiligen Tragabschnitts mit der Deckschicht vollständig auf der Oberseite der Gitterstruktur vorgenommen, so dass der jeweilige Tragabschnitt dann also seitens der Oberseite vollständig mit der Deckschicht abgedeckt ist. In vorteilhafter Weise ist der jeweilige Tragabschnitt somit auf der Oberseite entweder in bestimmten Bereichen gezielt abgedeckt oder vollständig beschichtet. Im letztgenannten Fall werden dadurch Gewebeirritationen auf der Oberseite vollständig verhindert, während bei der abschnittsweisen Beschichtung gezielt Kontaktbereiche mit Gewebe und/oder Unebenheiten des jeweiligen Tragabschnitts, wie beispielsweise Kanten oder Ähnliches, mithilfe der Deckschicht weicher gestaltet werden können.

Alternativ oder ergänzend dazu kann der jeweilige Tragabschnitt auf der Unterseite der Gitterstruktur an mindestens einem Teilabschnitt mit der jeweils einen Deckschicht beschichtet sein. Somit ist der jeweilige Tragabschnitt in diesem Fall an der Unterseite der Gitterstruktur abschnittsweise mit der Deckschicht versehen, wodurch der jeweilige Tragabschnitt also an der Unterseite abschnittsweise unbeschichtet ist. Der jeweilige Tragabschnitt könnte aber auch auf der Unterseite der Gitterstruktur vollständig mit der Deckschicht beschichtet sein, womit der jeweilige Tragabschnitt dann auf der Unterseite vollständig mit Deckschicht abgedeckt ist. In beiden Fällen wird dadurch an der Unterseite des Implantats zumindest abschnittsweise eine weichere Oberfläche verwirklicht, indem der jeweilige Tragabschnitt an der Unterseite entweder zumindest abschnittsweise oder vollständig mit der Deckschicht beschichtet ist. Hierdurch können Gewebeirritationen an der Unterseite des Implantats reduziert werden. Im Falle der abschnittsweisen Beschichtung des jeweiligen Tragabschnitts wird dieser dabei insbesondere gezielt in bestimmten Kontaktbereichen mit Gewebe und/oder an Unebenheiten, wie beispielsweise Kanten oder ähnlichem, beschichtet.

Die vorgenannten Weiterbildungen der Erfindung können dabei alternativ oder auch ergänzend verwirklicht sein, wodurch der jeweilige Tragabschnitt abschnittsweise oder vollständig auf der Oberseite beschichtet, abschnittsweise oder vollständig auf der Unterseite beschichtet oder sowohl abschnittsweise oder vollständig auf der Oberseite als auch abschnittsweise oder vollständig auf der Unterseite beschichtet sein kann.

Bei Kombination ist zudem eine Ausführung des Implantats denkbar, bei welcher der jeweilige Tragabschnitt vollständig mit der Deckschicht ummantelt ist. In diesem Fall ist der jeweilige Tragabschnitt dann also vollständig von der zugehörigen Deckschicht umschlossen, d.h. vollständig beschichtet.

Im Rahmen der Erfindung kann die Deckschicht porös ausgebildet sein. Dies hat den Vorteil, dass durch diese poröse Gestaltung eine besonders niedrige Härte der jeweiligen Deckschicht erreicht wird. Zum anderen wird hierdurch die Möglichkeit geschaffen, dass Gewebe in das Implantat einwachsen kann und eine Vaskularisierung möglich ist. In Weiterbildung dieser Ausführungsform ist der jeweilige Tragabschnitt dann zumindest abschnittsweise in die poröse Deckschicht eingebettet. In vorteilhafter Weise kann hierdurch in dem entsprechenden Bereich eine ebenmäßige, weiche Oberfläche verwirklicht werden.

Alternativ dazu kann die Deckschicht nicht porös ausgeführt sein. Dadurch kann in diesem Bereich eine sehr glatte, weiche Oberfläche realisiert werden, wodurch Gewebeirritationen weitestgehend vermindert sind. Durch den nicht porösen Aufbau der Deckschicht wird dabei ein Anwachsen von Geweben verhindert. Insbesondere entspricht hierbei eine jeweilige Strukturierung der Deckschicht einer Strukturierung, welche der jeweilige Tragabschnitt im Bereich der Beschichtung mit der Deckschicht aufweist. Dies hat den Vorteil, dass hierdurch eine Struktur des jeweiligen Tragabschnitts beibehalten wird.

Ganz besonders bevorzugt ist die geringere Härte der aufgebrachten Deckschicht dadurch erreicht, dass die aufgebrachte Deckschicht aus einem biokompatiblen Werkstoff hergestellt ist, welcher eine geringere Härte aufweist als der Werkstoff des jeweiligen Tragabschnitts. Bei der besonders bevorzugten Ausgestaltungsmöglichkeit der Erfindung, bei welcher der zumindest eine erste Tragabschnitt aus Polyetheretherketon und der zumindest eine zweite Tragabschnitt aus Polyethylen besteht, ist die aufgebrachte Deckschicht ebenfalls aus Polyethylen ausgestaltet. Dabei kann die Deckschicht dann nur im Bereich des zumindest einen ersten Tragabschnitts ausgestaltet oder auch zusätzlich an dem ebenfalls aus Polyethylen bestehenden, zweiten Tragabschnitt ausgebildet sein, um hier beispielsweise aufgrund einer porösen Gestaltung der Deckschicht eine noch weichere Oberfläche des Implantats zu erzeugen.

Vorteilhafte Ausführungsformen der Erfindung, die nachfolgend erläutert werden, sind in den Zeichnungen dargestellt. Es zeigen:
- Fig. 1 und 2: schematische Ansichten eines Implantats entsprechend einer ersten Ausführungsform;
- Fig. 3: eine schematische Ansicht eines Implantats gemäß einer zweiten Ausgestaltungsmöglichkeit;
- Fig. 4: eine schematische Darstellung eines Implantats gemäß einer dritten Ausführungsform;
- Fig. 5: eine schematische Ansicht eines Implantats gemäß einer vierten Ausgestaltungsmöglichkeit; und
- Fig. 6 bis 8: schematische Darstellungen von Abwandlungsmöglichkeiten der Implantate aus den Fig. 1 bis 5;

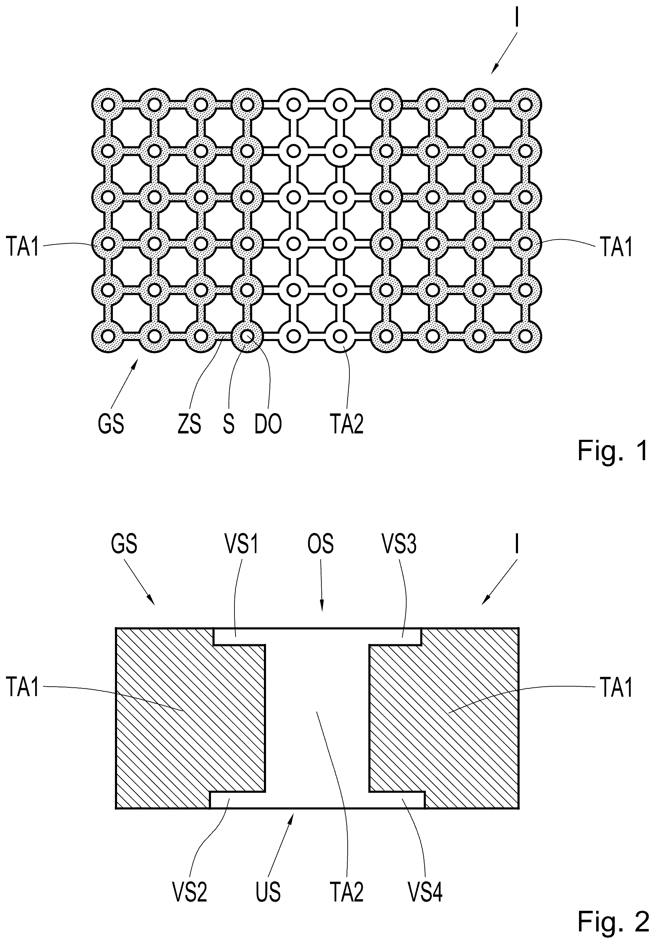

Aus den Fig. 1 und 2 gehen schematische Ansichten eines Implantats I hervor, welches für eine flächige Behandlung von Knochendefekten und hierbei insbesondere im Bereich des Thorax vorgesehen ist. Wie in Fig. 1 zu erkennen ist, weist das Implantat I eine Gitterstruktur GS auf, welche durch mehrere Tragabschnitte TA1 und TA2 gebildet ist. In der Gitterstruktur GS ist ein Tragabschnitt TA2 dabei zwischen zwei Tragabschnitten TA1 angeordnet, welche über den jeweils zwischenliegenden Tragabschnitt TA2 miteinander gekoppelt sind. Dazu ist der Tragabschnitt TA2 in der Gitterstruktur GS beidseitig mit den Tragabschnitten TA1 unter Ausbildung der flächigen Gitterstruktur GS verbunden.

Wie anhand von Fig. 1 zu erkennen ist, weisen die Tragabschnitte TA1 und TA2 jeweils eine Netzstruktur auf, indem sich der jeweilige Tragabschnitt TA1 bzw. TA2 aus ringförmigen Segmenten S und Zwischensegmenten ZS zusammensetzt, die als lineare Zwischenstücke gestaltet und über welche die ringförmigen Segmente S untereinander verbunden sind. Dabei bilden die ringförmigen Segmente S bei dem jeweiligen Tragabschnitt TA1 bzw. TA2 jeweils eine Durchgangsöffnung DO, in welcher je eine Knochenschraube zur Befestigung des Implantats I aufgenommen werden kann. Über die Knochenschrauben kann dabei eine knochenseitige Befestigung des Implantats I im Bereich des zu behandelnden Knochendefekts vorgenommen werden. Konkret können dabei im Bereich des Thorax Knochensegmente bzw. Knochenteile eines oder mehrerer Rippenknochen und/oder mehrere Rippenknochen untereinander verbunden werden, wobei das Implantat I dann über die Gitterstruktur GS eine entsprechende Stabilisierung herbeiführt.

Die beidseitig des Tragabschnitts TA2 liegenden Tragabschnitte TA1 bestehen jeweils aus einem ersten biokompatiblen Werkstoff in Form von Polyetheretherketon (PEEK), wobei der jeweilige Tragabschnitt TA1 dabei im Rahmen eines additiven Fertigungsverfahrens und hierbei insbesondere im Rahmen eines 3D-Drucks hergestellt worden ist. Hingegen ist der jeweils zwischenliegende und die beiden Tragabschnitte TA1 bei dem Implantat I miteinander verbindende Tragabschnitt TA2 aus einem zweiten biokompatiblen Werkstoff hergestellt, bei welchem es sich vorliegend um Polyethylen (PE) handelt.

Wie aus Fig. 2 hervorgeht, ist im Bereich der Verbindung des zwischenliegenden Tragabschnitts TA2 mit dem jeweils benachbart liegenden Tragabschnitt TA1 jeweils eine Überdeckung in einer Richtung vorgenommen, welche quer zu einer Oberseite OS und einer Unterseite US der Gitterstruktur GS verläuft. Dabei liegt die Oberseite der Unterseite US abgewandt, an welcher das Implantat I seitens des zu behandelnden Knochendefekts aufzulegen und zu befestigen ist. Um die jeweilige Überdeckung zu verwirklichen, ist der zwischenliegende Tragabschnitt TA2 an Enden, an welchen jeweils die Verbindung zu dem jeweils benachbart liegenden Tragabschnitt TA1 hergestellt ist, mit Verbindungssegmenten VS1 und VS2 bzw. VS3 und VS4 ausgestattet. Dabei sind diese Verbindungssegmente VS1 bis VS4 paarweise klammerartig vorstehend an dem zwischenliegenden Tragabschnitt TA2 ausgestaltet, wobei der Tragabschnitt TA2 dann im Bereich der jeweiligen Verbindung den jeweiligen Tragabschnitt TA1 mit den zugehörigen Verbindungssegmenten VS1 und VS2 bzw. VS3 und VS4 umgreift.

Die Verbindungen des Tragabschnitts TA2 mit den beidseitig liegenden Tragabschnitten TA1 sind vorliegend jeweils durch Stoffschluss realisiert, indem das den Tragabschnitt TA2 bildende Polyethylen im Rahmen eines Pressvorgangs unter Hitze gemeinsam mit dem die Tragabschnitte TA1 bildenden Polyetheretherketon (PEEK) verschmolzen worden ist. Dazu wurden die Tragabschnitte TA1 im Anschluss an ihre additive Fertigung einer Plasmabehandlung/Plasmaaktivierung unterzogen und anschließend in eine Negativform der Gitterstruktur GS eingelegt, wobei der zwischen den Tragabschnitten TA1 liegende Freiraum anschließend mit Polyethylen-Pulver befüllt wurde. Im Rahmen des Pressvorgangs bildet das Polyethylen dabei den zwischenliegenden Tragabschnitt TA2 und verbindet diesen zugleich stoffschlüssig mit den beidseitig liegenden Tragabschnitten TA1. Hierbei wird durch das Polyethylen eine nicht poröse Schicht gebildet.

Die Gitterstruktur GS des Implantats I ist flexibel gestaltet, wobei diese Flexibilität zum einen dadurch gegeben ist, dass die Zwischensegmente ZS bei dem jeweiligen Tragabschnitt TA1 bzw. TA2 Bewegungen der ringförmigen Segmente S zueinander ermöglichen. Diese Flexibilität der Gitterstruktur GS ist im Bereich des zwischenliegenden Tragabschnitts TA2 zudem noch erhöht, indem der zwischenliegende Tragabschnitt TA2 aufgrund seiner Herstellung aus Polyethylen eine geringere Steifigkeit aufweist als die beidseitig hierzu liegenden Tragabschnitte TA1. Grund hierfür ist die geringere Werkstoffsteifigkeit des Polyethylens im Vergleich zu dem Polyetheretherketon der beidseitig liegenden Tragabschnitte TA1. Durch diesen Aufbau der Gitterstruktur GS kann das Implantat I problemlos an gekrümmte Verläufe der Knochen im Bereich des zu behandelnden Knochendefekts angepasst werden. Zum anderen wird dadurch im befestigten Zustand des Implantats I eine gewisse Beweglichkeit zugelassen, um Bewegungen des Thorax aufgrund der Atmung oder von Bewegungen des Patienten zu erleichtern. Abgesehen von der gesteigerten Flexibilität der Gitterstruktur GS sorgt der zwischenliegende Tragabschnitt TA2 zudem aufgrund seiner niedrigeren Steifigkeit und der im Vergleich zum Polyetheretherketon geringeren Härte des Polyethylens auch für geringere Gewebeirritationen.

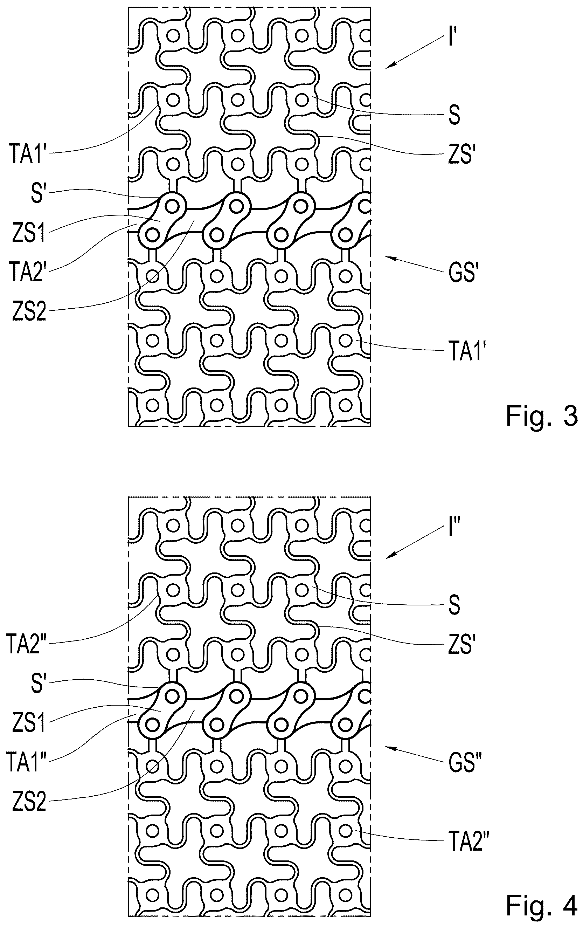

Fig. 3 zeigt eine Ansicht eines Teils eines weiteren Implantats I', welches dabei weitestgehend der vorhergehenden Variante nach den Fig. 1 und 2 entspricht. So umfasst dieses Implantat I' ebenfalls eine Gitterstruktur GS', welche durch mehrere Tragabschnitte TA1' und TA2' gebildet ist. In dieser Gitterstruktur GS' sind dabei additiv aus Polyetheretherketon hergestellte Tragabschnitte TA1' über jeweils einen zwischenliegenden Tragabschnitt TA2' aus Polyethylen miteinander verbunden. Im Unterschied zu dem Implantat I aus den Fig. 1 und 2 ist der zwischenliegende Tragabschnitt TA2' nun aber plattenartig hergestellt. Bei diesem plattenartigen Aufbau sind ringförmige Segmente S' einerseits über Zwischensegmente ZS1 paarweise miteinander verbunden und bilden hierdurch 8-förmige Gebilde, wobei diese Gebilde dabei dann über weitere, stegartige Zwischensegmente ZS2 untereinander unter Bildung des Tragabschnitts TA2' miteinander in Verbindung stehen. Ferner ist bei den beidseitig des jeweiligen Tragabschnitts TA2' liegenden Tragabschnitten TA1' eine gegenüber dem Implantat I aus Fig. 1 und 2 abweichende Netzstruktur gewählt, indem die Segmente S nun jeweils über nicht lineare, S-förmige Zwischensegmente ZS' untereinander verbunden sind. Ansonsten entspricht die Ausführungsform nach Fig. 3 der Variante nach den Fig. 1 und 2, so dass auf das hierzu Beschriebene Bezug genommen wird. Insbesondere ist dabei auch die Verbindung des zwischenliegenden Tragabschnitts TA2' mit dem jeweils benachbarten Tragabschnitt TA1' mit Überdeckung realisiert.

Hingegen ist bei dem in Fig. 4 gezeigten, erfindungsgemäßen Implantat I" im Vergleich zu der Variante nach Fig. 3 ein dahingehender, gespiegelter Aufbau gewählt, dass nun bei einer Gitterstruktur GS" ein jeweiliger, additiv aus Polyetheretherketon hergestellter Tragabschnitt TA1" den zu Fig. 3 beschriebenen plattenartigen Aufbau aufweist, während Tragabschnitte TA2" aus Polyethylen nun jeweils mit der zu Fig. 3 beschriebenen Netzstruktur versehen sind. Jeder der Tragabschnitte TA2" verbindet den jeweiligen Tragabschnitt TA1" dann in der Gitterstruktur GS" mit weiteren, plattenartigen Tragabschnitten aus Polyetheretherketon. Auch im Übrigen entspricht die Variante nach Fig. 4 der vorhergehenden Variante nach Fig. 3, so dass auf das hierzu Beschriebene Bezug genommen wird.

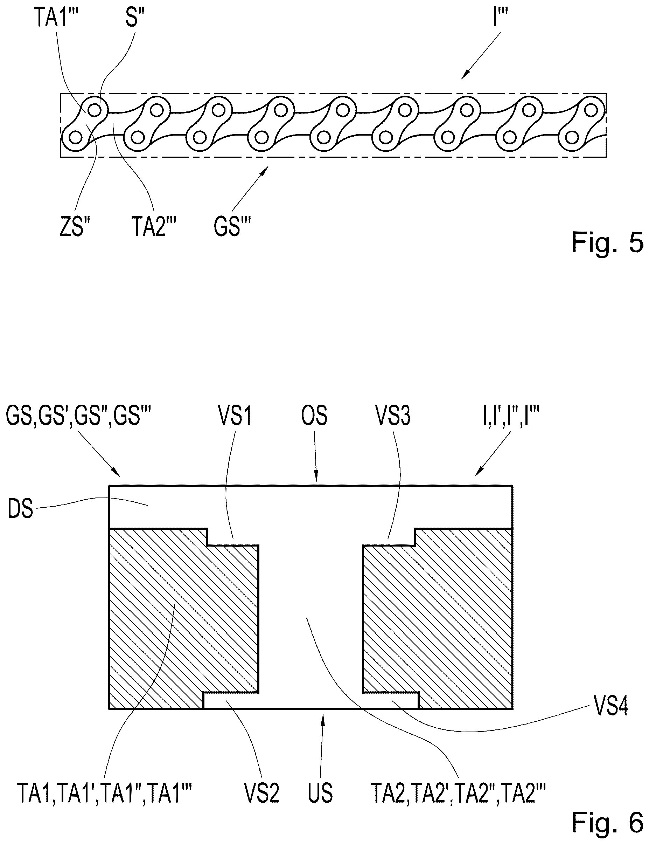

Des Weiteren geht Fig. 5 eine erfindungsgemäße Ausgestaltung eines Implantats I‴ hervor, wobei dieses Implantat I‴ dabei weitestgehend der Variante nach Fig. 3 entspricht. Unterschiedlich ist dabei, dass bei dem Implantat I‴ nun in einer Gitterstruktur GS‴ Tragabschnitte TA1‴ aus Polyetheretherketon (PEEK) als 8-förmige Gebilde ausgeführt sind, bei welchen ringförmige Segmente S" paarweise über je ein zwischenliegendes Zwischensegment ZS" miteinander verbunden sind. Untereinander sind die Tragabschnitte TA1‴ dann über zwischenliegende Tragabschnitte TA2‴ gekoppelt, welche im Vergleich zu den Tragabschnitten TA1‴ jeweils eine geringere Steifigkeit aufweisen, indem diese Tragabschnitte TA2‴ aus Polyethylen (PE) bestehen. Eine Verbindung des einzelnen Tragabschnitts TA2‴ mit den beidseitig liegenden Tragabschnitten TA1‴ ist dabei in analoger Weise zu der Variante zu den Fig. 1 und 2 vollzogen. Im Übrigen entspricht die Ausgestaltung nach Fig. 5 der Variante nach Fig. 3, so dass auf das hierzu Beschriebene Bezug genommen wird.

Schließlich zeigen die Fig. 6 bis 8 jeweils Abwandlungsmöglichkeiten der Implantate I, I', I" und I‴ aus den Fig. 1 bis 5. Bei der Abwandlungsmöglichkeit nach Fig. 6 sind die Tragabschnitte TA1 und TA2 bzw. TA1' und TA2' bzw. TA1" und TA2" bzw. TA1‴ und TA2‴ teilweise beschichtet, indem nun eine Deckschicht DS auf der Oberseite OS des jeweiligen Implantats I bzw. I' bzw. I" bzw. I‴ vorgesehen ist. Diese Deckschicht DS ist dabei aus Polyethylen hergestellt, wobei die Deckschicht DS dabei porös oder nicht porös ausgebildet sein kann.

Hintergrund für das Aufbringen der Deckschicht DS auf der Oberseite OS des jeweiligen Implantats I bzw. I' bzw. I" bzw. I‴ ist dabei, dass hierdurch insbesondere im Bereich der additiv hergestellten Tragabschnitte TA1 bzw. TA1' bzw. TA1" bzw. TA1‴ eine weichere Oberfläche zu erreichen ist. Denn die additive Herstellung der Tragabschnitte TA1 bzw. TA1' bzw. TA1" bzw. TA1‴ hat zur Folge, dass an dem jeweiligen Tragabschnitt TA1 bzw. TA1' bzw. TA1" bzw. TA1‴ eine raue Oberfläche und teilweise auch harte Kanten entstehen, was nach Platzierung des Implantats I bzw. I' bzw. I" bzw. I‴ im Körper des Patienten zu Gewebeirritationen führen kann. Darüber hinaus ist die Struktur des jeweiligen Tragabschnitts TA1 bzw. TA1' bzw. TA1" bzw. TA1‴ unter Umständen durch das Gewebe bzw. die Haut des Patienten hindurch spürbar, was ebenfalls in entsprechenden Irritationen resultieren kann. Dies ist nun dadurch reduziert, dass die Deckschicht DS aufgrund der Ausgestaltung aus Polyethylen eine im Vergleich zu den Tragabschnitten TA1 bzw. TA1' bzw. TA1" bzw. TA1‴ geringere Härte aufweist.

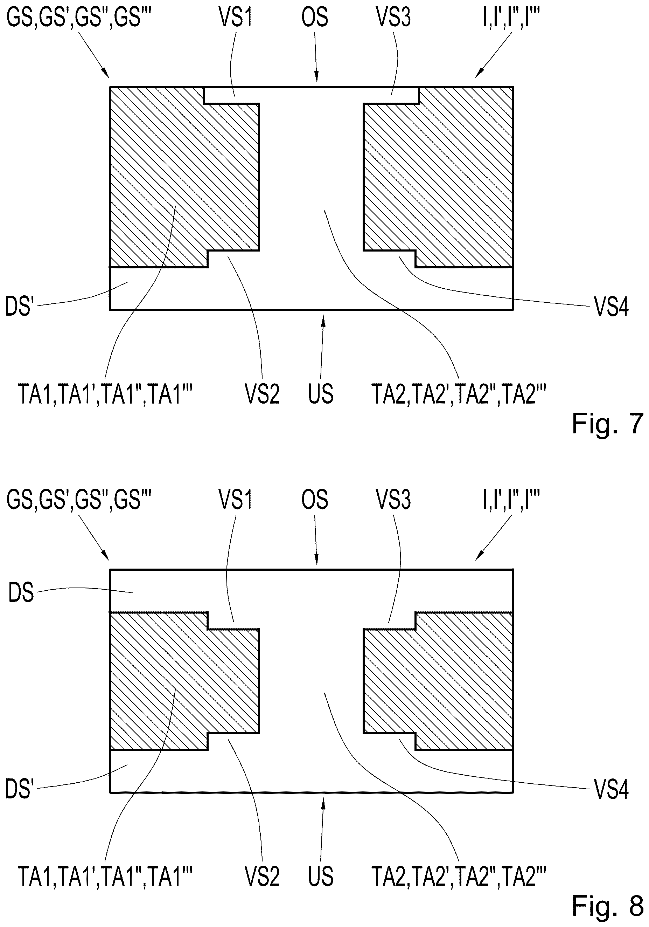

Bei der Abwandlungsmöglichkeit nach Fig. 7 ist eine Deckschicht DS' in analoger Weise zu der Abwandlungsmöglichkeit nach Fig. 6, allerdings dieses Mal auf der Unterseite US des jeweiligen Implantats I bzw. I' bzw. I" bzw. I‴ aufgebracht, um hier eine weichere Oberfläche zu realisieren.

Schließlich zeigt noch Fig. 8 eine weitere Abwandlungsmöglichkeit der Implantate I, I', I" und I‴ aus den Fig. 1 bis 5, wobei das jeweilige Implantat I, I', I" und I‴ nun sowohl auf der Oberseite OS mit der Deckschicht DS als auch auf der Unterseite US mit der Deckschicht DS' beschichtet ist. Dadurch ist die jeweilige Gitterstruktur GS bzw. GS' bzw. GS" bzw. GS‴ des jeweiligen Implantats I, I', I" und I‴ nun sandwichartig zwischen den Deckschichten DS und DS' eingefasst.

Mittels der erfindungsgemäßen Ausgestaltungen kann jeweils ein Implantat geschaffen werden, über welches eine zuverlässige flächige Behandlung eines Knochendefekts realisierbar ist, wobei bei Verwendung dieses Implantats zudem die Gefahr von Gewebeirritationen vermindert ist.

### Bezugszeichenliste

- I, I', I", I‴: Implantat
- GS, GS', GS", GS‴: Gitterstruktur
- TA1, TA2, TA1', TA2', TA1", TA2", TA1‴, TA2‴: Tragabschnitte
- S, S', S": Segmente
- ZS, ZS1, ZS2, ZS', ZS": Zwischensegmenten
- DO: Durchgangsöffnung
- OS: Oberseite
- US: Unterseite
- VS1, VS2, VS3, VS4: Verbindungsegmente
- DS, DS': Deckschicht

## Patentansprüche

1. Implantat (I; I'; I"; I‴) für eine flächige Behandlung eines Knochendefekts, insbesondere eines Knochendefekts im Bereich des Thorax oder des Craniums, umfassend eine flexible Gitterstruktur (GS; GS'; GS"; GS‴) mit einer Oberseite (OS) und einer der Oberseite (OS) abgewandt liegenden Unterseite (US), auf welcher eine knochenseitige Befestigung des Implantats (I; I'; I"; I‴) vorzunehmen ist, wobei die Gitterstruktur (GS; GS'; GS"; GS‴) mindestens einen ersten Tragabschnitt (TA1; TA1'; TA1"; TA1‴) aufweist, welcher aus einem ersten biokompatiblen Werkstoff besteht, **dadurch gekennzeichnet, dass** die Gitterstruktur (GS; GS'; GS"; GS‴) zusätzlich zu dem mindestens einen ersten Tragabschnitt (TA1; TA1'; TA1"; TA1‴)mindestens einen zweiten Tragabschnitt (TA2; TA2'; TA2"; TA2‴) aufweist, welcher über eine im Vergleich zu dem mindestens einen ersten Tragabschnitt (TA1; TA1'; TA1"; TA1‴) geringere Steifigkeit verfügt, indem der mindestens eine zweite Tragabschnitt (TA2; TA2'; TA2"; TA2‴) aus einem zweiten biokompatiblen Werkstoff hergestellt ist, welcher eine geringere Werkstoffsteifigkeit aufweist als der erste Werkstoff.

2. Implantat (I; I'; I"; I‴) nach Anspruch 1, **dadurch gekennzeichnet, dass** der mindestens eine zweite Tragabschnitt (TA2; TA2'; TA2"; TA2‴) in der Gitterstruktur (GS; GS'; GS"; GS‴) zwischen zumindest zwei ersten Tragabschnitten (TA1; TA1'; TA1"; TA1‴) angeordnet ist und die zumindest zwei ersten Tragabschnitte (TA1; TA1'; TA1"; TA1‴) miteinander koppelt.

3. Implantat (I; I'; I"; I‴) nach Anspruch 1 oder 2, **dadurch gekennzeichnet, dass** die Gitterstruktur (GS; GS'; GS"; GS‴) modular aufgebaut ist, indem der mindestens eine erste Tragabschnitt (TA1; TA1'; TA1"; TA1‴) und der mindestens eine zweite Tragabschnitt (TA2; TA2'; TA2"; TA2‴) aneinander befestigt sind, insbesondere durch eine stoffschlüssigen Verbindung zwischen dem mindestens einen ersten Tragabschnitt (TA1; TA1'; TA1"; TA1‴) und dem mindestes einen zweiten Tragabschnitt (TA2; TA2'; TA2"; TA2‴).

4. Implantat (I; I'; I"; I‴) nach Anspruch 3, **dadurch gekennzeichnet, dass** der mindestens eine zweite Tragabschnitt (TA2; TA2'; TA2"; TA2‴) im Bereich der jeweiligen Befestigung in einer quer zu der Oberseite (OS) und der Unterseite (US) verlaufenden Richtung den mindestens einen ersten Tragabschnitt (TA1; TA1'; TA1"; TA1‴) überdeckt.

5. Implantat (I; I'; I"; I‴) nach Anspruch 4, **dadurch gekennzeichnet, dass** der mindestens eine zweite Tragabschnitt (TA2; TA2'; TA2"; TA2‴) den mindestens einen ersten Tragabschnitt (TA1; TA1'; TA1"; TA1‴) im Bereich der jeweiligen Befestigung mit vorstehenden Verbindungssegmenten (VS1, VS2, VS3, VS4) beidseitig umgreift.

6. Implantat (I; I'; I"; I‴) nach wenigstens einem der vorhergehenden Ansprüche, **dadurch gekennzeichnet, dass** zumindest einer der Tragabschnitte (TA1, TA2; TA1', TA2'; TA1", TA2"; TA1‴, TA2‴) je eine Netzstruktur aufweist, welche durch miteinander über Zwischensegmente (ZS; ZS') verbundene, in sich geschlossene Segmente (S) gebildet ist.

7. Implantat (I'; I"; I‴) nach wenigstens einem der vorhergehenden Ansprüche, **dadurch gekennzeichnet, dass** zumindest einer der Tragabschnitte (TA1', TA2'; TA1", TA2"; TA1‴, TA2‴) plattenförmig gestaltet ist.

8. Implantat (I; I'; I"; I‴) nach wenigstens einem der vorhergehenden Ansprüche, **dadurch gekennzeichnet, dass** zumindest einer der Tragabschnitte (TA1, TA2; TA1', TA2'; TA1", TA2"; TA1‴, TA2‴), insbesondere der mindestens eine erste Tragabschnitt (TA1; TA1'; TA1"; TA1‴), mittels eines additiven Fertigungsverfahrens hergestellt ist.

9. Implantat (I; I'; I"; I"') nach wenigstens einem der vorhergehenden Ansprüche, **dadurch gekennzeichnet, dass** der zweite Werkstoff eine geringere Härte aufweist als der erste Werkstoff.

10. Implantat (I; I'; I"; I‴) nach wenigstens einem der vorhergehenden Ansprüche, **dadurch gekennzeichnet, dass** der erste Werkstoff ein Metall oder eine Metalllegierung, insbesondere Titan oder eine Titanlegierung, oder ein Kunststoff ist, insbesondere ein Polymer, bevorzugt ein Thermoplast und besonders bevorzugt Polyetheretherketon (PEEK).

11. Implantat (I; I'; I"; I‴) nach wenigstens einem der vorhergehenden Ansprüche, **dadurch gekennzeichnet, dass** der zweite Werkstoff ein Kunststoff ist, insbesondere ein Polymer, bevorzugt ein Thermoplast und besonders bevorzugt Polyethylen (PE).

12. Implantat (I; I'; I"; I‴) nach wenigstens einem der vorhergehenden Ansprüche, **dadurch gekennzeichnet, dass** auf zumindest einen der Tragabschnitte (TA1, TA2; TA1', TA2'; TA1", TA2"; TA1"', TA2‴), insbesondere auf den mindestens einen ersten Tragabschnitt (TA1; TA1'; TA1"; TA1‴), zumindest abschnittsweise eine Deckschicht (DS; DS') aufgebracht ist, welche im Vergleich zu dem jeweiligen Tragabschnitt (TA1; TA1'; TA1"; TA1‴) eine geringere Härte aufweist.

13. Implantat (I; I'; I"; I‴) nach Anspruch 12, **dadurch gekennzeichnet, dass** der jeweilige Tragabschnitt (TA1, TA2; TA1', TA2'; TA1", TA2"; TA1‴, TA2‴) auf der Oberseite (OS) der Gitterstruktur (GS; GS'; GS"; GS‴) zumindest an Teilabschnitten oder vollständig mit der Deckschicht (DS) beschichtet ist.

14. Implantat (I; I'; I"; I‴) nach Anspruch 12 oder 13, **dadurch gekennzeichnet, dass** der jeweilige Tragabschnitt (TA1, TA2; TA1', TA2'; TA1", TA2"; TA1‴, TA2"') auf der Unterseite (US) der Gitterstruktur (GS; GS'; GS"; GS‴) zumindest an Teilabschnitten oder vollständig mit der Deckschicht (DS') beschichtet ist.
